# EUROPEAN PATENT APPLICATION

(11) **EP 3 991 717 A1**
(43) Date of publication of application: **04.05.2022**
(21) Application number: 20205490.4
(22) Date of filing: 03.11.2020
(51) Int. Cl.: A61K 8/73, A61Q 19/08

(54) **ANTI-WRINKLE COSMETIC COMPOSITION**

(71) Applicant: SanderStrothmann GmbH, 49124 Georgsmarienhütte (DE)
(72) Inventor: STROTHMANN, Rene, 49186 Bad Iburg (DE); KNEIP, Thomas, 56220 Urmitz (DE)
(74) Representative: f & e patent

(57) **Abstract**

A cosmetic composition comprising three hyaluronic acids having different average molecular weight ranges being present in specific weight ratios is disclosed. The present invention achieves an improvement in wrinkle depth by providing sufficient moisturization of all levels of the human skin, while not causing or promoting skin irritations, allergic reactions and/or inflammations.

## Description

The present invention is directed to a cosmetic composition in form of a hydrogel. Said hydrogel comprises three different types of hyaluronic acid defined by their molecular weights, or their sodium and/or potassium salts, in specific weight ratios to each other. The present invention is also related to the use of such a composition for the reduction of wrinkles, preferably periocular wrinkles.

Hyaluronic acid (or hyaluronan according to more recent nomenclature) is a polymer of disaccharides, composed of D-glucuronic acid and N-acetyl-D-glucosamine, linked via alternating β-(1→4) and β-(1→3) glycosidic bonds.

Hyaluronic acid is naturally found in the human body. In the epidermis, hyaluronic acid content is especially high in proliferating basal regions. Histological findings suggest that basal layer keratinocytes contain intracellular hyaluronic acid, whereas extracellular hyaluronic acid prevails in upper epidermal layers. Extracellular hyaluronic acid is thought to maintain diffusion and to open up spaces to facilitate cell migration. The main source of hyaluronic acid in the dermis is fibroblasts with higher hyaluronic acid synthesis activity in the papillary dermis. The high flexibility of hyaluronic acid strands and their hydrophilicity enable these molecules to fill in any gaps within the extracellular matrix. The large hydrodynamic volume of hyaluronic acid is mainly explained by multiple hydrogen bonds between adjacent disaccharides but may also rely on the close interaction of hyaluronic acid with highly glycosylated proteoglycans. The resulting viscoelastic properties of hyaluronic acid in the dermis account for its support of tissue architecture and possibly function as a kind of 'hydraulic shock absorber' in the dermis.

However, human cells do not always produce hyaluronic acid efficiently lifelong. By age and following menopause, hyaluronic acid production declines in the skin, resulting in joint pain, mycofacial rigidity, loss of elasticity, ageing, dryness and development of wrinkles. Topically applied, hyaluronic acid can complement the deficiencies in endogenous hyaluronic acid and can contribute to reduce signs of skin ageing.

Cosmetic and pharmaceutical compositions comprising hyaluronic acid have previously been described in the art. US 2019/0314263 A1 discloses cosmetic compositions comprising a chemically crosslinked hyaluronic acid and a hyaluronic acid having a molecular weight of about 50 kDa. US 10,660,420 B1 discloses a topical composition comprising two different hyaluronic acids, one with a preferred average molecular weight from 1.200 kDa to 3.000 kDa and one with a preferred average molecular weight from 8 kDa to 50 kDa, in combination with polyaspartic acid. US patent 8,426,383 B2 discloses a topical composition comprising two types of hyaluronic acids having average molecular weights from 500 kDa to 3.000 kDa and 0.5 kDa to 100 kDa, respectively, in combination polysaccharides extracted from root bark of *Ulmus davidiana.* WO 2019/046678 discloses pharmaceutical compositions for the treatment of keloids, comprising hyaluronic acids with average molecular weights ranging from 4 to 5.000 kDa. US 2019/0282487 A1 discloses cosmetic compositions comprising two different hyaluronic acids, one with a preferred average molecular weight of about 2.100 kDa and one with a preferred average molecular weight of not more than 10 kDa.

Compositions disclosed in the prior art have several disadvantages. Hyaluronic acids mostly having high average molecular weights, i.e. more than 2.200 kDa, permeate not or only slightly into the upper skin layers and are quickly washed away by the consumer. Compositions comprising mainly hyaluronic acids in this molecular weight range therefor lack any long-term effects.

However, hyaluronic acids mostly having very low average molecular weights, i.e. less than 80 kDa permeate deeply into the skin, but have the disadvantage to promote inflammatory reactions. Consumers using compositions comprising such hyaluronic acids may therefore encounter skin irritations or inflammations.

Additionally, compositions known in the art do not contain amounts of hyaluronic acids of different molecular weight ranges, which sufficiently permeate into all skin layers, thereby not providing evenly balanced hydration of the skin.

Compositions disclosed in the prior art further have the disadvantage that they require rather extensive efforts during formulation. These include several formulation steps, heating or rapid agitation of (parts of) the composition or the pre-preparation of several parts of the composition, which then need to be combined, often also under heating and rapid agitation. Such formulations are therefore rather ineffective to formulate, thereby being both uneconomic and energy-consuming.

It was therefore an object of the present invention to provide an anti-wrinkle cosmetic composition, which can be easily prepared, i.e. without using elevated temperatures and high agitation for a prolonged time, and which can provide excellent or even improved moisturization to all levels of the skin and significant reduction of wrinkles, particularly periocular wrinkles. The latter effects should be achieved while not causing any irritations or allergic reactions to the skin, regardless of age and skin type of the consumer using the composition.

This object is met by a cosmetic composition in form of a hydrogel, comprising (i) at least one hyaluronic acid having an average molecular weight from 1800 kDa to 2200 kDa, or its sodium or potassium salt(s), (ii) at least one hyaluronic acid having an average molecular weight from 1000 kDa to 1800 kDa, or its sodium or potassium salt(s), and (iii) at least one hyaluronic acid having an average molecular weight from 100 kDa to 400 kDa, or its sodium or potassium salt(s), wherein components (i) and (ii) are present in a weight ratio from 5:1 to 1:5, preferably from 3:1 to 1:3, more preferably from 1.5:1 to 1:1.5, and wherein components (i) and (iii) are present in a weight ratio from 10:1 to 1:1, preferably 7:1 to 1.5:1, more preferably 5:1 to 2:1.

The term "hydrogel" as used herein refers to a water-based gel received by adding hygroscopic polymers into an aqueous base. The mixture thickens until becoming an aqueous gel. This effect is achieved by interactions of the polymer chains, namely hydrogen bonding, entanglement of two or more polymer chains or covalent bonding. A skilled person will acknowledge that the formation of covalent bonds between several polymer chains, e.g. by crosslinking agents, result in chemically crosslinked polymers. However, the hyaluronic acids contained in the hydrogel according to the present invention are preferably not chemically crosslinked. The inventive hydrogel is thus preferably formed by hydrogen bonds and entanglement of the different hyaluronic acid chains.

Unless otherwise stated, all percentages mentioned herein are weight-percentages, based on the total weight of the composition, and all ratios mentioned herein are weight-ratios (w/w).

### Hyaluronic acids

Within the context of the present invention, three different types of hyaluronic acid are defined by their average molecular weights. The term "high molecular weight hyaluronic acid" refers to hyaluronic acids having an average molecular weight from more than 1800 kDa to 2200 kDa, "low molecular weight hyaluronic acid" refers to hyaluronic acids having an average molecular weight from 1000 kDa to 1800 kDa, and "very low molecular weight hyaluronic acid" refers to hyaluronic acids having an average molecular weight from 100 kDa to 400 kDa. When referring to "hyaluronic acid(s)", the present invention also encompasses the free acid form as well as the corresponding sodium and/or potassium salts, and mixtures thereof. In case any substance or ingredient mentioned herein is followed by the term INCI in parentheses, the substances or ingredients name refers to its systematic name according to the International Nomenclature of Cosmetic Ingredients.

The average molecular weight of the hyaluronic acids present in the composition may be determined by any technique known in the art, including viscometry, size exclusion chromatography, or gel electrophoresis using a monodisperse hyaluronic acid standard.

The total amount of hyaluronic acids (i) to (iii) present in the cosmetic composition preferably ranges from 0.1 wt.-% to 10 wt.-%, more preferably 0.2 to 5 wt.-%, even more preferably 0.3 wt.-% to 2.5 wt.-%, most preferred 0.4 wt.-% to 1 wt.-%, based on the total weight of the cosmetic composition. If their sodium or potassium salts are used, the given amounts refer to the hyaluronic acid anion, wherein the counter ion is not included in the calculation.

The inventors have surprisingly found that the combination of the above-mentioned hyaluronic acids in the specific weight-ratios results in a well-balanced distribution of hyaluronic acids throughout all layers of the skin, thereby evenly moisturizing the upper, middle and lower skin layers. The high molecular weight hyaluronic acid slightly permeates into the upper skin layers, resulting in a lesser tendency of being simply washed away by the consumer. Furthermore, the very low molecular weight hyaluronic acid has an average molecular weight, which is sufficiently low to allow it to permeate deeply into the skin, but not so low that it would promote any inflammatory or allergic reactions.

### Chelating agents

The cosmetic composition further preferably comprises a low molecular weight compound having at least two, preferably three or four carboxylic acid groups, or at least one carboxylic acid group and at least one keto, aldehyde or hydroxy group, which further is referred to as a "chelating agent" or as a "chelator". With "low molecular weight" in reference to this chelator a molecular weight of less than 1000, preferably less than 800, even more preferred less than 500 or even less than 400 g per mol (g mol⁻¹) is meant, referring to the free acid groups, counter-ion(s) not included in the calculation. It is preferred that at least one type of such a chelator is present in the inventive composition, preferably in form of its sodium or potassium salt, or mixtures thereof.

Such chelating agents have a double function in the cosmetic composition: on one hand they are able to form complexes with trace metals, which might otherwise adversely affect stability or appearance of the composition, on the other hand the acid groups are able to form hydrogen bonds with the free hydroxy groups of the hyaluronic acids resulting in a "net" of hyaluronic acid chains, connected via the chelator molecules by hydrogen bonds. By forming said hydrogen bond based (thus flexibly bonds-building and solving) "net", also the other components of the cosmetic composition are kept evenly distributed within the composition, even after application to the skin within the several skin layers. Due to the flexibility of the hydrogen bonds all compounds which have a size small enough to permeate into the skin, can pass the skin barrier and can re-build hydrogen bonds in the skin.

Examples for suitable chelators are ethylenediamine tetraacetic acid, nitrilotriacetic acid and L-glutamic acid-N,N-diacetic acid, or their sodium or potassium salts, respectively, as well as dicarboxylic acids with at least two C atoms, preferably naturally occurring dicarboxylic acids with C2 to C10 like oxalic acid, malonic acid, succinic acid, glutaric acid, adipinic acid, pimelic acid, suberic acid, acelaic acid or sebacic acid, or unsaturated dicarboxylic acids like fumaric acid or maleic acid, tricarboxylic acids like citric acid, aconitic acid, isocitric acid, or naturally occurring acids with at least one carboxylic acid and at least one keto group, at least one aldehyde group or at least one additional hydroxy group like e.g. pyruvic acid, glyoxylic acid, oxo-glutaric acid, oxaloacetic acid, oxalosuccinic acid or malic acid, or their sodium or potassium salts without being restricted to the mentioned.

Most preferred, the chelator(s) is/are, selected from ethylenediamine tetraacetic acid (EDTA), nitrilotriacetic acid, sodium or potassium salts thereof, and L-glutamic acid-N,N-diacetic acid (its Na salt INCI TETRASODIUM GLUTAMATE DIACETATE), and mixtures thereof. The most preferred chelator is TETRASODIUM GLUTAMATE DIACETATE, so that it is preferred that at least this chelator is present in the composition.

Chelating agents may be present in the cosmetic composition in a total amount of from 0.004 wt.-% to 2 wt.-%, preferably from 0.01 to 0.625 wt.-%, more preferred from 0.02 wt.-% to 0.25 wt.-%, based on the total weight of the cosmetic composition.

The weight ratio of the total content of hyaluronic acids to the total content of chelator(s), if present, preferably ranges from 25:1 to 5:1 (w/w), more preferably 20:1 to 8:1 (w/w) and even more preferably 15:1 to 10:1 (w/w).

### Thickening, stabilizing and film-forming agents

The cosmetic composition may also comprise one or more polysaccharide(s). Said polysaccharides may serve as thickening agents and/or as stabilizing agents for the hyaluronic acids. These polysaccharide(s) may be at least one selected from dextran, agarose, xylan, xanthan, mannan, carrageenan, alginate, gelatin, arabinogalactan, cellulose, methyl cellulose, ethyl cellulose, hydroxypropyl methyl cellulose, carboxymethyl cellulose, hydroxyethyl starch, chitosan, and mixtures thereof. Preferably, the polysaccharide(s) is/are selected from xanthan, carrageenan, arabinogalactan, and mixtures thereof. If present, polysaccharides may be present in the cosmetic composition in a total amount of from 0.01 wt.-% to 10 wt.-%, preferably from 0.03 to 7.5 wt.-%, more preferred from 0.075 wt.-% to 2.5 wt.-%, based on the total weight of the composition.

The ratio of the total content of hyaluronic acids, or its sodium or potassium salts, to the total content of polysaccharide(s), if present, preferably ranges from 10:1 to 1:2 (w/w), more preferably 6:1 to 1:1.5 (w/w) and even more preferably 4:1 to 1:1 (w/w).

### Further polymeric compounds

The cosmetic composition may further comprise at least one polymeric compound different from polysaccharides. Preferred polymeric compounds may be selected from the group consisting of polyethylene glycol, polypropylene glycol, polyethylene oxide, polypropylene oxide, polyglutamate, polylysine, polysialic acid, polyvinyl alcohol, polyacrylate, polymethacrylate, polyacrylamide, polymethacrylamide, polyvinyl pyrrolidone, polyoxazoline, polyiminocarbonate, polyamino acid, hydrophilic polyester, polyamide, polyurethane, polyurea, copolymers comprising acrylic acid, methacrylic acid, hydroxymethylacrylic acid or hydroxyethylacrylic acid monomers, and mixtures thereof. More preferably, polymeric compounds may be selected from the group consisting of polyacrylate, polymethacrylate, copolymers comprising acrylic acid, methacrylic acid, hydroxymethylacrylic acid or hydroxyethylacrylic acid monomers, and mixtures thereof. Most preferred, the cosmetic composition comprises at least two of the before-mentioned polymeric compounds different from polysaccharides.

Polymeric compounds different from polysaccharides may be present in the cosmetic composition in a total amount of from 0.05 wt.-% to 5 wt.-%, preferably 0.1 wt.-% to 3 wt.-%, more preferably 0.2 wt.-% to 1 wt.-%.

### Humectants

The composition may contain one or more humectants in a total amount of from 0.01 wt.-% to 20 wt.-%, preferably 0.5 wt.-% to 10 wt.-%, more preferably from 2 wt.-% to 8 wt.-%. Suitable humectants include glycols and polyols, urea and its derivatives, and sugars. Suitable glycols, polyols and sugars may comprise glycerin, 1,2-propandiol, 1,2-butandiol, 1,2-pentandiol, 1,2-hexandiol, 1,2-octandiol, 1,2-decandiol, 2-methyl-1,3-propandiol, xylitol, sorbitol, glucose, fructose, honey, hydrogenated honey, inositol, maltose, mannitol, maltitol, sucrose, and/or xylose. A particularly preferred humectant is glycerin.

### Preservatives

The composition may comprise at least one preservative in a total amount of from 0.1 wt.-% to 5 wt.-%, preferably from 0.5 wt.-% to 3 wt.-%, more preferred from 0.8 wt.-% to 1.5 wt.-%, in order to suppress microbial growth and therefore improve the stability of the composition. Preferred preservatives are phenoxyethanol and ethylhexylglycerin.

In a preferred embodiment, the cosmetic composition comprises at least one of glycerin, phenoxyethanol and/or ethylhexylglyerin, more preferably at least two of them, most preferred all three compounds.

In case all three compounds are present, they may be present in a total amount from 0.1 wt.-% to 20 wt.-%, preferably from 1 wt.-% 10 wt.-%, more preferred from 3 wt.-% to 8 wt.-%.

### Oils

The cosmetic composition preferably comprises at least one oil. The term "oil" in this context may refer to any natural or synthetic oils, including linear or branched fatty alcohols, glycols or esters having six or more carbon atoms. Silicones and silicone oils are excluded from this definition, as these compounds are considered as undesired ingredients, which are discussed further below. The at least one oil may preferably be present in the composition in a total amount from 0.01 wt.-% to 10 wt.-%, more preferred from 0.01 wt.-% to 3 wt.-% and even more preferred from 0.01 wt.-% to 2 wt.-%, based on the total weight of the composition. Particularly preferred oils are esters of linear and branched fatty acids with linear or branched fatty alcohols, wherein independently the linear or branched fatty acid and the linear or branched fatty alcohol may contain 6 to 16 carbon atoms, preferably 6 to 12 carbon atoms, more preferably 6 to 10 carbon atoms, with linear or branched fatty alcohols having 6 to 16 carbon atoms, preferably 6 to 12 carbon atoms, more preferably 8 to 12 carbon atoms.

### Active ingredients

To further improve the anti-aging effects achieved by the above-described combination of hyaluronic acids, the composition may comprise at least one active ingredient selected from skin lighteners, antioxidants and skin conditioning agents, and mixtures thereof.

Skin lighteners are capable of promoting an even tone of the skin by lightening dark spots and further preventing UV-induced darkening of the skin. Examples of skin lighteners are 4-butylresorcinol, phenylethyl resorcinol (INCI) as well as inorganic pigments like titanium dioxide and zinc oxide. A preferred skin lightener is phenylethyl resorcinol (INCI), which is commercially available e.g. as SymWhite^{®} 377 from Symrise. Skin lighteners may be present in the cosmetic composition in a total amount from 0.1 wt.-% to 2 wt.-%, preferably 0.15 wt.-% to 1 wt.-%, more preferred 0.2 wt.-% to 0.5 wt.-%, based on the total weight of the cosmetic composition.

Antioxidants reduce the amount of oxidative stress by scavenging reactive oxygen species including superoxide radicals, hydroxyl radicals and hydrogen peroxide. Antioxidants may comprise naturally occurring antioxidants and synthetic antioxidants. Naturally occurring antioxidants may comprise vitamins, including their derivatives, like vitamins A (referring to retinol, retinol esters, retinal and retinoic acid), C (ascorbic acid) and E (tocopherol), tocopherol acetate, tocopherol ferulate, ascorbyl palmitate, magnesium ascorbyl phosphate and retinyl palmitate, and non-vitamin-based antioxidants, like sclareolide, a natural product derived from *Salvia sclarea,* coenzyme Q10 (ubiquinone), lycopene, green tea extract, silymarin, resveratrol, pycnogenol, genistein or niacinamide. Synthetic antioxidants may for example comprise propyl gallate (PG), idebenone and tertiary butylhydroquinone (TBHQ). A particularly preferred antioxidant is sclareolide.

Antioxidants may be present in the cosmetic composition in a total amount from 0.01 wt.-% to 2 wt.-%, preferably 0.05 wt.-% to 1 wt.-%, more preferred 0.1 wt.-% to 0.5 wt.-%, based on the total weight of the cosmetic composition.

Skin conditioning agents reduce skin roughness and may further support reduction of wrinkle depth and/or formation. A particularly preferred skin-conditioning agent is acetyl hexapeptide-8 (INCI), commercially available under the trade name Argireline^{®} by Lipotec. This particular oligomeric peptide is capable of achieving similar effects on wrinkle depth as botulinum toxin A (a bacterial toxin known in the art to be injected into the skin in order to reduce wrinkles), but with the advantage that acetyl hexapeptide-8 (INCI) is non-toxic. It therefore does not require injections to be carried out by qualified personnel, but can be easily incorporated into a cosmetic composition, which can be topically applied by the consumer.

Without wishing to be bound by theory, the inventors believe that both acetyl hexapeptide-8 (INCI) and botulinum toxin A target the same protein complex responsible for modulating muscle contraction, known as the SNARE complex. Both compounds slightly destabilize the complex, resulting in the vesicle not being able to release neurotransmitters. The consequence is a relaxation of the respective muscles, thereby decreasing the formation of lines and wrinkles as well as decreasing the depth of existing ones.

If present, acetyl hexapeptide-8 (INCI) may be present in a total amount from 0.001 wt.-% to 0.1 wt.-%, preferably 0.002 wt.-% to 0.07 wt.-%, more preferably 0.003 wt.-% to 0.05 wt.-%, based on the total weight of the cosmetic composition.

The total amount of skin conditioning agents may range from 0.001 wt.-% to 1 wt.-%, preferably 0.002 wt.-% to 0.7 wt.-%, more preferably 0.003 wt.-% to 0.5 wt.-%, based on the total weight of the cosmetic composition.

If present, the ratio of the total amount of skin lighteners to the total amount of hyaluronic acids may range from 20:1 to 1:100 (w/w), preferably from 5:1 to 1:33 (w/w), more preferably from 1.25:1 to 1:5 (w/w). If present, the ratio of the total amount of antioxidants to the total amount of hyaluronic acids may range from 20:1 to 1:1.000 (w/w), preferably from 5:1 to 1:100 (w/w), more preferably from 1.67:1 to 1:25 (w/w). If present, the ratio of the total amount of skin conditioning agents to the total amount of hyaluronic acids may range from 10:1 to 1:10.000 (w/w), preferably from 3.5:1 to 1:2.500 (w/w), more preferably from 1.67:1 to 1:333 (w/w).

### Preferred combinations of ingredients

It is preferred that the cosmetic composition comprises, in addition to the high, low and very low molecular weight hyaluronic acids, at least one of the above-mentioned chelators, at least one polysaccharide, a combination of chelator(s) and polysaccharide(s), or a combination of chelator(s) and at least one of the active ingredients mentioned above.

In a particularly preferred embodiment, the cosmetic composition comprises the following components:
i. at least one hyaluronic acid having a molecular weight from 1800 kDa to 2200 kDa, or its sodium or potassium salt(s), preferably in an amount of from 0.01 wt-% to 5 wt.-%,
ii. at least one hyaluronic acid having a molecular weight from 1000 kDa to 1800 kDa, or its sodium or potassium salt(s), preferably in an amount of from 0.01 wt.-% to 5 wt.-%,
iii. at least one hyaluronic acid having a molecular weight from 100 kDa to 400 kDa, or its sodium or potassium salt(s), preferably in an amount of from 0.01 wt.-% to 3 wt.-%,
iv. at least one chelator (preferably the tetrasodium salt of L-glutamic acid-N,N-diacetic acid), preferably in an amount of from 0.01 wt.-% to 1 wt.-%,
v. at least one polysaccharide selected from xanthan, carrageenan, arabinogalactan, and mixtures thereof, preferably in a total amount of these three from 0.01 wt.-% to 2 wt.-%, and
vi. at least one of the group: skin lighteners comprising phenylethyl resorcinol, antioxidants comprising sclareolide, and/or skin conditioning agents comprising acetyl hexapeptide-8 (INCI),
wherein given weight-percentage numbers are based on the total weight of the cosmetic composition,
wherein components (i) and (ii) are present in a ratio from 5:1 to 1:5, preferably from 3:1 to 1:3, more preferably from 1.5:1 to 1:1.5, and
wherein components (i) and (iii) are present in a ratio from 10:1 to 1:1, preferably 7:1 to 1.5:1, more preferably 5:1 to 2:1.

The cosmetic composition may additionally comprise ingredients known in the art suitable for a cosmetic composition, such as antioxidants, humectants, plant extracts, vitamins and skin protecting agents and further anti-aging actives.

### Undesired ingredients

The inventive composition is preferably free of several compounds. Free in this context means that, despite efforts taken to avoid the presence of an undesired compound, an undesired compound or ingredient may be present in an amount of less than 0.2 wt-%, preferably less than 0.1 wt.-%, or less than 0.05 wt.-% more preferred less than 0.01 wt.-% and most preferred 0 wt.-%, based on the total weight of the composition.

In order to avoid skin irritations due to drying out of the skin, it is preferred that the cosmetic composition is free of ethanol and polyethylene glycols.

Due to the not finally assessed impact on the environment, an inventive composition is preferably free of silicone oils and more preferably free of silicones. A skilled person will acknowledge that silicone oils are at 20 °C liquid silicones. Silicones in general are linear, cyclic or crosslinked, oligo- or polymeric compounds, which may comprise the monomer units R₂SiO, RSiO_{1.5}, R₃SiO_{0.5} and/or SiO₂, wherein R may be H, alkyl, alkenyl or aryl.

Parabens, particularly methyl-, ethyl-, propyl-, butyl- and isopropylparaben, are known for their properties as preservatives. However, parabens have a reputation for having estrogenic activity. Furthermore, there have been reports of parabens accumulating in the animal and potentially also in the human body. Therefore, the cosmetic composition is preferably free of parabens.

### Physical properties

The cosmetic composition may have a density at 20 °C from 0.9 g/cm³ to 1.1 g/cm³, preferably from 0.95 g/cm³ to 1.05 g/cm³, more preferred from 0.97 g/cm³ to 1.03 g/cm³, a pH value from 5 to 7.5, preferably from 5.5 to 6.8, and/or a viscosity (20 °C, spindle RV 3, 10 rpm) from 3.000 mPa·s to 15.000 mPa·s, preferably from 3.500 mPa·s to 14.000 mPa·s, more preferred from 4.000 mPa·s to 13.200 mPa·s.

### Example

The following example is meant to further illustrate the beneficial effects of the cosmetic composition and therefore not intended to limit the present invention in any way.

A hydrogel according to the table below was prepared by mixing the specified ingredients under mediumly paced agitation and letting the composition swell. No heating or pre-preparation of parts of the composition were required.

Said hydrogel was applied 2 to 3 times per week over a period of four weeks to the left half of the face of twenty female subjects aged 31-67. All subjects had sensitive skin.

| | |
|---|---|
| Sodium hyaluronate having an average molecular weight from more than 1800 kDa to 2200 kDa | about 0.3 wt.-% |
| Sodium hyaluronate having an average molecular weight from 1000 kDa to 1800 kDa | about 0.3 wt.-% |
| Sodium hyaluronate having an average molecular weight from 100 kDa to 400 kDa | about 0.15 wt.-% |
| Humectants (glycerin, caprylyl glycol) | about 5 wt.-% |
| Polysaccharides (Xanthan gum, carrageenan, arabinogalactan) | about 0.5 wt.-% |
| Acetyl hexapeptide-8 (INCI) | About 0.006 wt.-% |
| Oil (propylheptyl caprylate) | about 6 wt.-% |
| Minors: polymers (sodium polyacrylate, hydroxyethyl acrylate/sodium acryloyl dimethyl taurate copolymer) preservatives (phenoxyethanol, ethylhexylglycerin) chelators (L-glutamic acid-N,N-diacetic acid, tetrasodium salt) | about 1.7 wt.-% |
| Water | ad 100 wt.-% |

After the test period, none of the test subjects encountered allergic reactions, skin irritations or inflammations.

## Claims

1. A cosmetic composition in form of a hydrogel, comprising:
i. at least one hyaluronic acid having an average molecular weight of more than 1800 kDa to 2200 kDa, or its sodium or potassium salt(s),
ii. at least one hyaluronic acid having an average molecular weight from 1000 kDa to 1800 kDa, or its sodium or potassium salt(s), and
iii. at least one hyaluronic acid having an average molecular weight from 100 kDa to 400 kDa, or its sodium or potassium salt(s),
wherein components (i) and (ii) are present in a weight ratio from 5:1 to 1:5, preferably from 3:1 to 1:3, more preferably from 1.5:1 to 1:1.5, and
wherein components (i) and (iii) are present in a weight ratio from 10:1 to 1:1, preferably 7:1 to 1.5:1, more preferably 5:1 to 2:1.

2. The cosmetic composition according to claim 1, wherein the total amount of hyaluronic acids (i) to (iii), or their sodium or potassium salts, ranges from 0.1 wt.-% to 10 wt.-%, preferably 0.2 to 5 wt.-%, more preferably 0.3 wt.-% to 2.5 wt.-%, most preferred 0.4 wt.-% to 1 wt.-%, based on the total weight of the composition.

3. The cosmetic composition according to any preceding claims, further comprising at least one chelator compound having at least two, preferably three or four carboxylic acid groups, or at least one carboxylic acid group and at least one keto, aldehyde or hydroxy group, a sodium or potassium salts thereof, and mixtures thereof, wherein said compound has a molecular weight of less than 1000 g per mol.

4. The cosmetic composition according to claim 3, wherein the chelator(s) is/are, selected from ethylenediamine tetraacetic acid, nitrilotriacetic acid and L-glutamic acid-N,N-diacetic acid, sodium or potassium salts thereof, and mixtures thereof, wherein preferably at least the sodium salt of L-glutamic acid-N,N-diacetic acid (INCI TETRASODIUM GLUTAMATE DIACETATE) is present.

5. The cosmetic composition according to any preceding claims, further comprising at least one polysaccharide selected from dextran, agarose, xylan, xanthan, mannan, carrageenan, alginate, gelatin, arabinogalactan, cellulose, methyl cellulose, ethyl cellulose, hydroxypropyl methyl cellulose, carboxymethyl cellulose, hydroxyethyl starch, chitosan, and mixtures thereof.

6. The cosmetic composition according to claim 4, wherein the polysaccharide(s) is/are selected from xanthan, carrageenan, arabinogalactan, and mixtures thereof.

7. The cosmetic composition according to any of the preceding claims, further comprising an active ingredient selected from skin lighteners, antioxidants and skin conditioning agents, and mixtures thereof, wherein preferably skin conditioning agents comprise acetyl hexapeptide-8 (INCI).

8. The cosmetic composition according to claim 7, wherein the ratio of the total content of hyaluronic acids, or its sodium or potassium salts, to
(a) the total content of skin lighteners, if present, ranges from 5:1 to 1:3 (w/w), preferably from 4:1 to 1:2 (w/w), more preferably from 3:1 to 1:1 (w/w), or
(b) the total content of antioxidants, if present, ranges from 7:1 to 1:2 (w/w), preferably from 5:1 to 1:1 (w/w), more preferably from 4:1 to 2:1 (w/w), or
(c) the total content of skin conditioning agents, if present, ranges from 0.01:1 to 0.2:1 (w/w), preferably from 0.02:1 to 0.1:1 (w/w), more preferably from 0.045:1 to 0.075:1 (w/w).

9. The cosmetic composition according to claims 3 to 8, wherein the ratio of the total content of hyaluronic acids, or its sodium or potassium salts, to the total content of chelator(s) ranges from 25:1 to 5:1 (w/w), preferably 20:1 to 8:1 (w/w), more preferably 15:1 to 10:1 (w/w).

10. The cosmetic composition according to claims 5 to 9, wherein the ratio of the total content of hyaluronic acids, or its sodium or potassium salts, to the total content of polysaccharide(s) ranges from 10:1 to 1:2 (w/w), preferably 6:1 to 1:1.5 (w/w), more preferably 4:1 to 1:1 (w/w).

11. The cosmetic composition according to any preceding claims, comprising:
i. at least one hyaluronic acid having a molecular weight from more than 1800 kDa to 2200 kDa, or its sodium or potassium salt(s), preferably in an amount of from 0.01 wt-% to 5 wt.-%,
ii. at least one hyaluronic acid having a molecular weight from 1000 kDa to 1800 kDa, or its sodium or potassium salt(s), preferably in an amount of from 0.01 wt.-% to 5 wt.-%,
iii. at least one hyaluronic acid having a molecular weight from 100 kDa to 400 kDa, or its sodium or potassium salt(s), preferably in an amount of from 0.01 wt.-% to 3 wt.-%,
iv. at least one chelator, preferably comprising the tetrasodium salt of L-glutamic acid-N,N-diacetic acid, preferably in an amount of from 0.01 wt.-% to 1 wt.-%,
v. at least one polysaccharide selected from xanthan, carrageenan, arabinogalactan, and mixtures thereof, preferably in a total amount of these three from 0.01 wt.-% to 2 wt.-%, and
vi. at least one of the group: skin lighteners comprising phenylethyl resorcinol, antioxidants comprising sclareolide, and/or skin conditioning agents comprising acetyl hexapeptide-8 (INCI),
wherein given weight-percentage numbers are based on the total weight of the composition,
wherein components (i) and (ii) are present in a ratio from 5:1 to 1:5, preferably from 3:1 to 1:3, more preferably from 1.5:1 to 1:1.5, and
wherein components (i) and (iii) are present in a ratio from 10:1 to 1:1, preferably 7:1 to 1.5:1, more preferably 5:1 to 2:1.

12. The cosmetic composition according to any preceding claims, further comprising at least one of glycerin, phenoxyethanol and/or ethylhexylglyerin, preferably at least two of them, more preferably all three compounds.

13. The cosmetic composition according to any preceding claims, further comprising at least one polymeric compound different from polysaccharides, selected from the group consisting of polyethylene glycol, polypropylene glycol, polyethylene oxide, polypropylene oxide, polyglutamate, polylysine, polysialic acid, polyvinyl alcohol, polyacrylate, polymethacrylate, polyacrylamide, polymethacrylamide, polyvinyl pyrrolidone, polyoxazoline, polyiminocarbonate, polyamino acid, hydrophilic polyester, polyamide, polyurethane, polyurea, copolymers comprising acrylic acid, methacrylic acid, hydroxymethylacrylic acid or hydroxyethylacrylic acid monomers, and mixtures thereof, preferably selected from the group consisting of polyacrylate, polymethacrylate, copolymers comprising acrylic acid, methacrylic acid, hydroxymethylacrylic acid or hydroxyethylacrylic acid monomers, and mixtures thereof.

14. The cosmetic composition according to any preceding claims, further comprising at least one compound selected from esters of linear or branched fatty acids having 6 to 16 carbon atoms, preferably 6 to 12 carbon atoms, more preferably 6 to 10 carbon atoms, with linear or branched fatty alcohols having 6 to 16 carbon atoms, preferably 6 to 12 carbon atoms, more preferably 8 to 12 carbon atoms.

15. Use of a cosmetic composition according to any preceding claims for the reduction of wrinkles, preferably periocular wrinkles.
